# EUROPEAN PATENT SPECIFICATION

(11) **EP 0 822 841 B1**
(45) Date of publication and mention of the grant of the patent: **22.01.2003**
(21) Application number: 96912753.9
(22) Date of filing: 15.04.1996
(51) Int. Cl.: A61M 5/142, A61M 39/02

(54) **BIORETENTIVE FILTERED INFUSION CATHETER**
INFUSIONSKATHETER MIT BIORETENTIVEM FILTER
CATHETER A PERFUSION FILTREE PAR BIORETENTEUR

(30) Priority: 28.04.1995 US 430959
(43) Date of publication of application: 11.02.1998
(73) Proprietor: MEDTRONIC, INC., Minneapolis, Minnesota 55432-3576 (US)
(72) Inventor: ELSBERRY, Dennis, D., Plymouth, MN 55442 (US)
(74) Representative: Hughes, Andrea Michelle
(86) International application number: US9605162
(87) International publication number: WO96033756

(56) References cited:
- WO-A-94/05351
- DE-A- 3 641 107
- GB-A- 2 061 749
- US-A- 3 640 269
- US-A- 4 021 353

## Description

The present invention relates generally to apparatus for preventing microbial contamination of drug infusion sites; and more particularly relates to such methods and apparatus implementing the use of an implanted bioretentive filter.

Implantable, continuous infusion has been shown to be an effective medication delivery method because it maintains continuous therapeutic drug delivery, improves patient quality of life, reduces rates of infection, and is more cost effective than traditional methods. However, the chronic infusion of drugs through permanently implanted catheters connected to either catheter access ports or implanted infusion pumps equipped with catheter access ports still creates significant risk to the patient from microbial infections. Conventional methods and apparatus directed to minimizing microbial infection focus on external bioretentive filters placed within the hypodermic needle used to deliver the drug. Such conventional methods are deficient, however, in that there is still a substantial risk of infection from the introduction of the hypodermic needle through the patient's skin to access the catheter access port, as each access through the skin provides an opportunity for bacterial infection.

Typically, the risk results in microbial infection at a rate of 5-15%. However, the probability of infection increases with the number of accesses into the catheter to administer the infusion therapy, and further escalates where preservative-free drugs or drugs which inherently support microbial growth are used. Such drugs may include peptides, polypeptides, proteins, glycoproteins, lipoproteins, oligonucleotides, oligonucleosides, hormonal agents, and/or other biologically derived trophic agents. Further, a particularly significant application of the invention is penetration of the blood/brain area. Such procedures usually are accompanied by an even greater rate of infection.

DE-A-36 41 107 discloses an arrangement for the subcutaneous introduction of drugs etc. whereby the drug is injected into a catheter by a needle. A filter layer may be provided in an inlet opening.

US-A-5,137,529 discloses a similar subcutaneously implantable injection port provided with a filter barrier.

As explained in more detail below, the present invention overcomes the above-noted and other shortcomings of prior apparatus and methods for preventing microbial infection of drug infusion sites.

Generally, the invention is directed to incorporating within a catheter infusion path a fluid conduit with an implanted bioretentive filter which prevents the microbial contamination of the target site to receive the infused drug.

According to the present invention, there is provided an implantable bioretentive filter assembly to filter fluid passing from an implanted fluid source to a delivery location in a patient's body, comprising:
a fluid conduit having a first end configured to be placed in fluid communication with said implanted fluid source and a second end configured to be placed in fluid communication with said delivery location within said patient's body, said fluid conduit formed of a material implantable within the patient's body; and characterised by
a cylindrical tubular bioretentive filter element retained within said fluid conduit to filter all fluid passing between said first end and said second end; said filter element having an exterior surface sealingly coupled to said conduit at a first end of said filter element and having an interior surface closed to fluid flow at a second end of said filter element, such that said fluid from said implanted fluid source passes through said tubular filter element from said interior surface to said exterior surface.

According to a second aspect, there is provided a fluid delivery system for delivering fluid from a fluid source implanted in a patient's body at a first location to a delivery point at a second location in said patient's body comprising:
a fluid source; and such a filter assembly.

A first embodiment is configured so that a cylindrically-shaped bioretentive filter inserted into the catheter's lumen prevents passage of microbial agents during the infusion process. In a particularly preferred embodiment, the filter is fabricated in a cylindrical shape having sufficient surface area for filtration to occur without adverse interruption of flow. As an example of physical size, the outside diameter of the filter assembly would probably be in the order of 0.5 to 1.0 mm, and 2 to 5 cm in length. The filter may be fabricated from one or several filter mediums having known compatibility with the drugs prescribed for infusion delivery. One example of such a filter would be one made of polyvinylidiene fluoride and polysulfones having pore sizes in the range from about 0.10 to about 0.22 micrometers. Such a suitable material is available from Amicon and is marketed under the name microporous hollow fiber.

A second embodiment of the present invention comprises the in-line insertion of a filtered fluid conduit with previously implanted infusion catheters to produce a bioretentive barrier between the point of infusion access and the target infusion site. Such a device would typically include a connector suitable for mating with either a permanently implanted catheter access port or an infusion pump at a first location, and a connector adapted for joining to an existing implanted infusion catheter at a second location downstream of the filter member. Again, the bioretentive filter is preferably cylindrical in shape, and is inserted into the lumen of the filtering device so as to create a bioretentive barrier for microbial agents. Filtering devices suitable for permanent implantation comprise known biocompatible and biostable materials, such as silicone elastomer, fluoroelastomer, fluoropolymer, titanium, and titanium alloys.

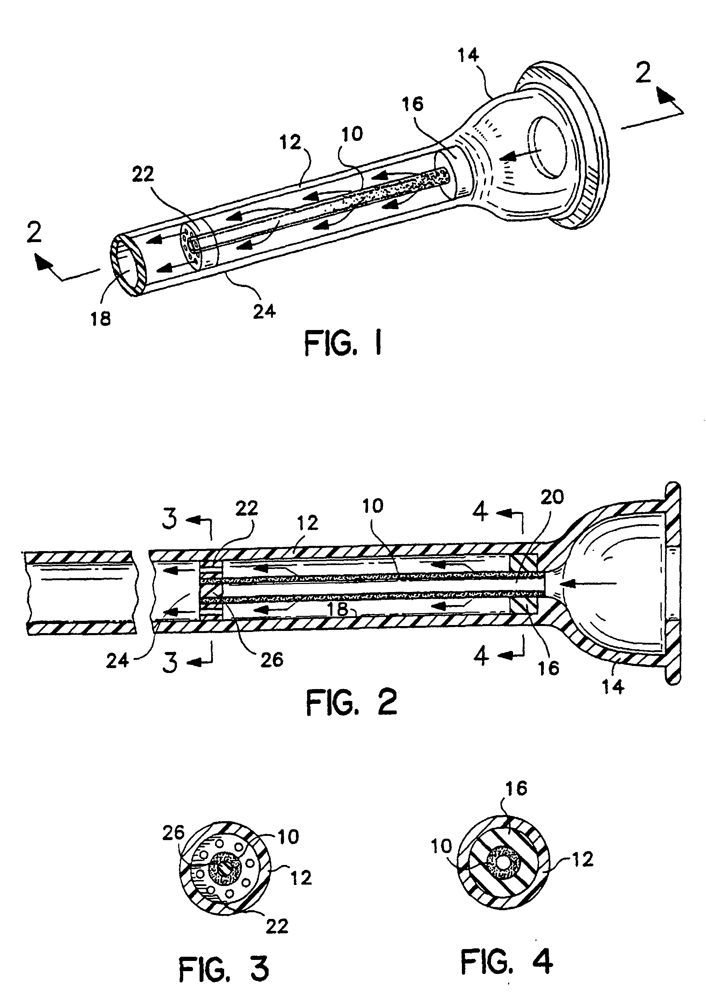

The two embodiments of the present invention broadly outlined above are shown in Figs. 1-6. As is evident from the drawings, and as is more fully described below, both embodiments generally are described as having a fluid flow within a fluid conduit from one point to another along a path from the interior of a cylindrical filter toward the exterior through the filter medium. One of ordinary skill in the art with the benefit of this disclosure will undoubtedly appreciate and understand that other filtering devices are possible, but are not within the scope of this patent, which have an inverted fluid flow path as compared to the one described herein. All that is necessary to do that is to reverse the orientation of the entire assembly relative to the fluid flow path. Such an embodiment may require, for example, additional structural spacers to be inserted within the filter to maintain its central passageway. The depicted embodiment is far preferable in that respect. However, under certain circumstances, it may be desirable to reverse the relative positions of the filters and seals in such a manner to allow filtered drug to flow from the lumen of the filter to allow outside catheter diameter reduction to access small target sites (such as spinal intrathecal space, intracerebroventricular access, specific brain nuclei, etc.).

FIG. 1 is an illustration of an exemplary bioretentive filtered infusion catheter in accordance with the present invention, depicted in a three-dimensional view.

FIG. 2 is an illustration of the bioretentive filtered infusion catheter shown in FIG. 1, depicted in a cross-sectional view.

FIG. 3 is an illustration of the bioretentive filtered infusion catheter shown in FIG. 1, depicted in a cross-sectional view taken along the line 3-3 shown in FIG. 2.

FIG. 4 is an illustration of the bioretentive filtered infusion catheter shown in FIG. 1, depicted in a cross-sectional view taken along the line 4-4 shown in FIG. 2.

FIG. 5 is an illustration of a bioretentive filtered fluid conduit in accordance with the present invention, depicted in a cross-sectional view.

FIG. 6 is an illustration of the bioretentive filtered fluid conduit shown in FIG. 5, depicted in a cross-sectional view.

FIGS. 1-4 show a bioretentive filter 10 incorporated into the body of a catheter or fluid conduit 12. Filter 10 is placed near a first catheter or fluid conduit end 14 adapted and configured to connect to either an access port (such as the Model 8501 Catheter Access Port manufactured by Medtronic, Inc., of Minneapolis, Minnesota) or an implanted infusion pump (such as the one used in the SynchroMedTM Infusion System developed and marketed by Medtronic, Inc.). Catheter 12 will be formed of a conventional biocompatible material as known to the art which is suitable for implantation with the body.

In the depicted exemplary preferred embodiment, cylindrical seal ring 16 seals the annular space between an inside catheter surface 18 and the filter 10 to direct flow into the filter's central core 20 and outward through the cylindrical wall of the filter 10. The solid retention block or seal ring 16 may not be required if the filter 10 is attached completely about the inner wall 18 of the catheter. Preferably, an annular seal 22 distal to the first catheter connection end 14 is perforated to allow flow of the filtered drug through the remainder of conduit 12 to the target site. However, the perforated retention block or seal 22 may not be necessary, in which case the filter assembly would essentially represent merely a bag. The core 20 of the cylindrical filter 10 at the filter end 24 opposite the catheter connection end 14 may comprise either a solid plug 26, or additional filter material.

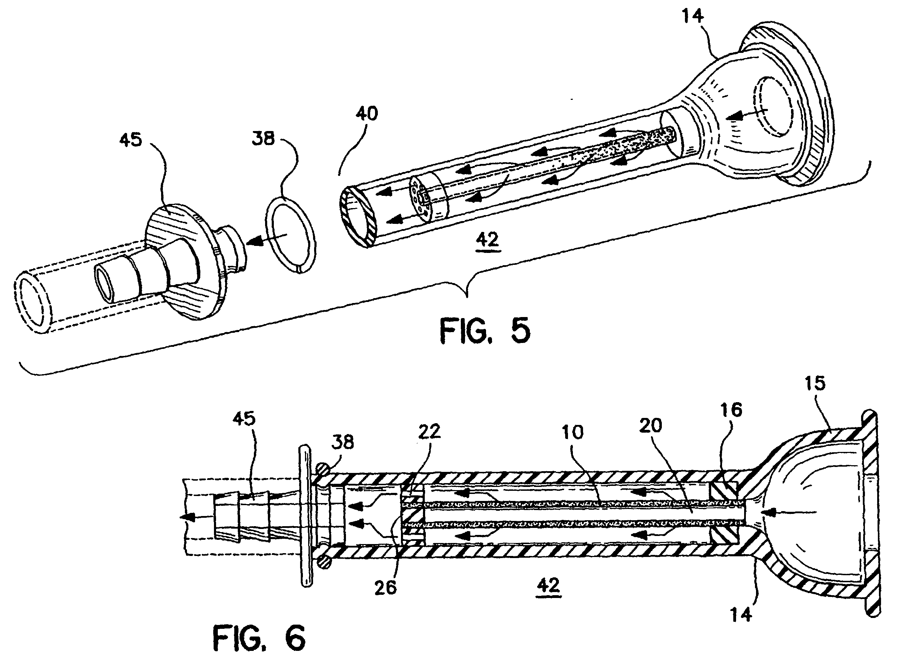

A second embodiment of the present invention is shown in FIGS. 5-6. The assembly comprises a fluid conduit 42 generally cylindrical in shape, having on one end 14 a molded connector 15 for mating with an access port or infusion pump, and on the other end 40 a fluted male connector 45 fabricated from a rigid material (i.e., for example, titanium, titanium alloys, or fluoropolymer) for mating with the implanted catheter to be retrofitted. Other configurations of mating connectors may be incorporated for facilitating coupling to selected devices. The mating of the male connector 45 to the rest of the assembly preferably is accomplished as shown with a metal compression ring 38. Of course other suitable means for attaching the male connector 45, such as polysiloxane adhesive or overmolding processes, may be used.

Although the preferred embodiment of this invention has been described hereinabove in some detail, it should be appreciated that a variety of embodiments will be readily available to persons utilizing the invention for a specific end use. Again, the description of the apparatus of this invention is not intended to be limiting on this invention, but is merely illustrative of the preferred embodiment of this invention. Additional objects, features and advantages of the present invention will become apparent by referring to the above description of the invention in connection with the accompanying drawings.

## Claims

1. An implantable bioretentive filter assembly (10) to filter fluid passing from an implanted fluid source to a delivery location in a patient's body, comprising:
a fluid conduit (12) having a first end (14) configured to be placed in fluid communication with said implanted fluid source and a second end (24) configured to be placed in fluid communication with said delivery location within said patient's body, said fluid conduit formed of a material implantable within the patient's body; and **characterised by**
a cylindrical tubular bioretentive filter element (10) retained within said fluid conduit to filter all fluid passing between said first end and said second end; said filter element having an exterior surface sealingly coupled (16) to said conduit at a first end of said filter element and having an interior surface (26) closed to fluid flow at a second end of said filter element, such that said fluid from said implanted fluid source passes through said tubular filter element from said interior surface to said exterior surface.

2. The filter assembly of claim 1, wherein said fluid conduit (12) comprises a portion of a catheter, said catheter extending to said delivery location.

3. A fluid delivery system for delivering fluid from a fluid source implanted in a patient's body at a first location to a delivery point at a second location in said patient's body comprising:
a fluid source; and a filter assembly as claimed in any of claims 1 or 2.

4. The fluid delivery system of claim 3, wherein said fluid source comprises an implanted access port.

5. The fluid delivery system of claim 3, wherein said fluid source comprises an infusion pump.

## Patentansprüche

1. Implantierbare Bioretentions-Filterbaueinheit (10) zum Filtern von Fluid, das sich von einer implantierten Fluidquelle zu einer Abgabe- bzw. Versorgungsstelle in einem Patientenkörper bewegt, mit:
einer Fluidleitung (12), die ein erstes Ende (14), das so konfiguriert ist, daß es in eine Fluidverbindung mit der implantierten Fluidquelle gebracht werden kann, und ein zweites Ende (24), das so konfiguriert ist, daß es in eine Fluidverbindung mit der Abgabestelle im Patientenkörper gebracht werden kann, aufweist, wobei die Fluidleitung aus einem in den Patientenkörper implantierbaren Werkstoff gebildet ist; und **gekennzeichnet durch**
ein zylindrisches, rohrförmiges Bioretentions-Filterelement (10), das in der Fluidleitung gehalten wird, um das gesamte zwischen dem ersten Ende und dem zweiten Ende sich bewegende Fluid zu filtern; wobei das Filterelement eine äußere Oberfläche, die an einem ersten Ende des Filterelements mit der Leitung dicht verbunden ist (16), und eine innere Oberfläche (26), die am zweiten Ende des Filterelements für eine Fluidströmung verschlossen ist, aufweist, so daß sich das Fluid von der implantierten Fluidquelle von der inneren Oberfläche zu der äußeren Oberfläche **durch** das rohrförmige Filterelement bewegt.

2. Filterbaueinheit nach Anspruch 1, bei der die Fluidleitung (12) einen Abschnitt eines Katheters aufweist, der zu der Abgabestelle verläuft.

3. Fluidversorgungs- bzw. abgabesystem zur Abgabe von Fluid von einer Fluidquelle, die an einer ersten Stelle in einem Patientenkörper implantiert ist, an einen Abgabepunkt an einer zweiten Stelle in dem Patientenkörper, mit:
einer Fluidquelle; und einer Filterbaueinheit nach einem der Ansprüche 1 oder 2.

4. Fluidabgabesystem nach Anspruch 3, bei dem die Fluidquelle einen implantierten Zugangsanschluß aufweist.

5. Fluidversorgungssystem nach Anspruch 3, bei dem die Fluidquelle eine Infusionspumpe aufweist.

## Revendications

1. Ensemble de filtre de biorétention implantable (10) destiné à filtrer des fluides passant d'une source de fluide implantée dans un site d'administration dans le corps d'un patient, comprenant :
un conduit fluidique (12) ayant une première extrémité (14) configurée de manière à être mise en communication fluidique avec ladite source de fluide implantée et une deuxième extrémité (24) configurée de manière à être mise en communication fluidique avec ledit site d'administration dans ledit corps du patient, ledit conduit fluidique étant formé d'un matériau implantable dans le corps du patient ; et **caractérisé par**
un élément de filtre de biorétention tubulaire cylindrique (10) retenu dans ledit conduit fluidique pour filtrer tout le fluide passant entre ladite première extrémité et ladite deuxième extrémité ; ledit élément de filtre ayant une surface extérieure accouplée de manière hermétique (16) audit conduit au niveau d'une première extrémité dudit élément de filtre et ayant une surface intérieure (26) fermée à l'écoulement fluidique au niveau d'une deuxième extrémité dudit élément de filtre, de sorte que ledit fluide provenant de ladite source de fluide implantée passe à travers ledit élément de filtre tubulaire depuis ladite surface intérieure jusqu'à ladite surface extérieure.

2. Ensemble de filtre selon la revendication 1, dans lequel ledit conduit fluidique (12) comprend une portion d'un cathéter, ledit cathéter s'étendant jusqu'audit site d'administration.

3. Système d'administration de fluide pour administrer du fluide d'une source de fluide implantée dans le corps d'un patient au niveau d'un premier site à un point d'administration au niveau d'un deuxième site dans ledit corps du patient, comprenant :
une source de fluide ; et un ensemble de filtre tel que revendiqué dans l'une quelconque des revendications 1 ou 2.

4. Système d'administation de fluide selon la revendication 3, dans lequel ladite source de fluide comprend un orifice d'accès implanté.

5. Système d'administration de fluide selon la revendication 3, dans lequel ladite source de fluide comprend une pompe à perfusion.
